(19) Europäisches Patentamt
European Patent Office
Office européen des brevets

(11) Veröffentlichungsnummer: **0 192 925**
**B1**

(12) # EUROPÄISCHE PATENTSCHRIFT

(45) Veröffentlichungstag der Patentschrift: **12.07.89**

(51) Int. Cl.⁴: **C 08 B 37/08**, A 61 K 7/48

(21) Anmeldenummer: **86100081.8**

(22) Anmeldetag: **04.01.86**

(54) **Kosmetische Mittel auf der Basis von quaternären Chitosanderivaten, neue quaternäre hydroxypropyl-substituierte Chitosanderivate sowie Verfahren zu ihrer Herstellung.**

(30) Priorität: **22.01.85 DE 3501891**

(43) Veröffentlichungstag der Anmeldung: **03.09.86 Patentblatt 86/36**

(45) Bekanntmachung des Hinweises auf die Patenterteilung: **12.07.89 Patentblatt 89/28**

(84) Benannte Vertragsstaaten: **AT BE CH DE FR GB IT LI LU NL SE**

(56) Entgegenhaltungen: **EP-A-0 115 574**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Lang, Günther, Dr., Auf der Roten Erde 10 B, D-6107 Reinheim 5 (DE)**
Erfinder: **Wendel, Harald, Grabengasse 3, D-6105 Ober- Ramstadt (DE)**
Erfinder: **Konrad, Eugen, Mecklenburger Strasse 101, D-6100 Darmstadt (DE)**

**EP 0 192 925 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft kosmetische Mittel zur Behandlung von Haaren oder der Haut mit einem Gehalt an neuen makromolekularen quaternären, vom Chitosan abgeleiteten Verbindungen in einer geeigneten Kosmetikgrundlage.

Die Erfindung betrifft weiterhin die neuen quaternären Chitosanderivate sowie Verfahren zu ihrer Herstellung.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationaktiver Polymere in solchen kosmetischen Mitteln zahlreiche Vorteile ergibt; die Entwirrung der Haare sowie deren Behandlung wird erleichtert, und weiterhin wird dem Haar Sprungkraft und Glanzwirkung verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 2 627 419.

In gleicher Weise wie bei der Mehrzahl der kationaktiven Polymere mit quaternären Gruppierungen ergibt Chitosan ebenfalls häufig den Nachteil, daß es mit den anionaktiven oberflächenaktiven Agentien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Durch Einsatz von quaternären Chitosanderivaten gemäß unserer eigenen EP-A 0 115 574 anstelle von Chitosansalzen lassen sich die meisten der vorstehend erwähnten Nachteile vermeiden. Die aus der EP-A 0 115 574 bekannten Chitosanderivate neigen jedoch bei wiederholter Anwendung, wenn auch weniger stark als die üblicherweise in Haarbehandlungsmitteln verwendeten kationischen Polymere (beispielsweise kationische Cellulosederivate vom Polymer® JR-Typ), so doch merklich zur Ansammlung auf den Haaren, wodurch diese belastet werden und rasch nachfetten. Hierdurch erhält das Haar einen unerwünschten schmierigen und fettigen Griff sowie ein ungepflegtes Aussehen. Weiterhin ist die Herstellung der dort beschriebenen quaternären Chitosanderivate sehr kostenintensiv, da das für die Herstellung dieser Chitosanderivate erforderliche Glycidol (2,3-Epoxy-1-propanol) ein teurer, großtechnisch nicht hergestellter und zudem hydrolyseempfindlicher Rohstoff ist.

Aufgabe der Erfindung ist es deshalb, Mittel zur Verfügung zu stellen, mit denen sich die vorstehend genannten Nachteile - insbesondere die starke Belastung der Haare bei wiederholter Anwendung - vermeiden lassen.

Bei der Fortführung der Untersuchungen mit Chitosan und den davon abgeleiteten Verbindungen wurde nunmehr gefunden, daß bestimmte quaternäre Chitosanderivate die vorstehend aufgeführten Nachteile nicht aufweisen.

Mit diesen quaternären Chitosanderivaten lassen sich somit kosmetische Mittel zur Behandlung von Haaren oder der Haut herstellen, die sich durch überraschend vorteilhafte Eigenschaften auszeichnen und die dadurch gekennzeichnet sind, daß sie in einer geeigneten Kosmetikgrundlage eine quaternäre makromolekulare, vom Chitosan abgeleitete polymere Verbindung der allgemeinen Formel I

$$HO[C_6H_{11-m}NO_4(R^1)_m(R^2)_n(R^3)_q]_pH, \qquad (I)$$

wobei

m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

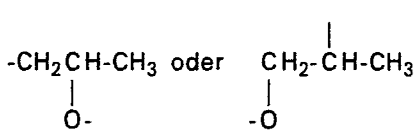

darstellt und $R^3$ ein zweiwertiger Rest

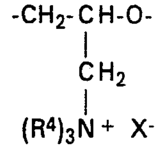 oder 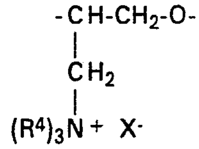

mit $R^4 = C_1$- bis $C_4$-Alkyl und X = Cl, Br, J oder $CH_3SO_4$ ist, enthalten.

Die erfindungsgemäßen, quaternäre Chitosanderivate der Formel I enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Mittel zur Festigung der Frisur, Waschlotionen, Fönlotionen, Haarkuren, Mittel gegen Kopfschuppen, Mittel zur permaneten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtswässer, Rasierwässer, Feuchthaltecremes, Coldcremes, Körperlotionen, Sonnenschutzmittel oder auch Make-up-Präparate wie Schminkcremes und Rouges.

Der Gehalt der erfindungsgemäßen kosmetischen Mittel an den neuen Chitosanderivaten der Formel I liegt zweckmäßig bei 0,05 bis 10 Gew.-%, vorzugsweise bei 0,05 bis 3,0 Gew.-%.

Die kosmetischen Mittel gemäß der vorliegenden Erfindung können zusätzlich zu dem neuen Chitosanderivat der Formel I zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemäßen kosmetischen Mittel weisen in bevorzugter Weise einen pH-Wert von 2 bis 11 auf und können in Form wäßriger, alkoholischer oder wäßrig-alkoholischer Zubereitungen, z. B. mit einem Alkohol mit 1 bis 4 Kohlenstoffatomen, als Lösungen, als Cremes, als Gele, als Dispersionen oder als Emulsionen vorliegen. Ebenfalls ist es möglich, diese Mittel in Form der alkoholischen oder wäßrig-alkoholischen Lösung mit Hilfe eines Zerstäubers bzw. anderer geeigneter Sprühvorrichtungen oder im Gemisch mit üblichen unter Druck verflüssigten Treibgasen als Aerosolhaarspray aus einem Druckbehälter zu versprühen.

Wenn es sich bei den erfindungsgemäßen kosmetischen Mitteln um Mittel zur Festigung der Frisur, wie flüssige Haarfestiger oder Haarsprays, handelt, dann liegen sie üblicherweise als wäßrig-alkoholische Lösungen vor, die durch einen Gehalt an quaternären Chitosanderivaten der vorstehend genannten Formel I gekennzeichnet sind. Hierbei können die quaternären Chitosanderivate selbst als filmbildendes bzw. festigendes Harz eingesetzt werden. Es können jedoch auch zusätzlich andere filmbildende natürliche oder synthetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden z. B. Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen bzw. die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung. Die Mittel weisen dann insbesondere einen pH-Wert zwischen 6 und 8 auf. Solche Mittel zur Festigung der Frisur enthalten üblicherweise filmbildende Polymere in einer Gesamtmenge von etwa 0,05 bis 3,0 Gew.-%. Enthalten die Mittel neben den quaternären Chitosanderivaten der Formel I noch andere filmbildende Polymere, so reduziert sich der Gehalt an quaternären Chitosanderivaten entsprechend.

Als Alkohole kommen insbesondere die für kosmetische Zwecke üblicherweise verwendeten niederen Alkohole wie Ethanol und Isopropanol in Betracht.

Die erfindungsgemäßen Mittel zur Festigung der Frisur können weiterhin die üblichen Zusätze wie beispielsweise Parfümöl, Bakterizide oder Fungizide, kämmbarkeitsverbessernde Substanzen usw. enthalten.

Die erfindungsgemäßen Mittel zur Festigung der Frisur können gegebenenfalls durch einen Gehalt an kosmetischen Farbstoffen das Haar gleichzeitig färben oder tönen. Derartige Präparate sind u.a. als Farbfestiger oder Tönungsfestiger im Handel bekannt. Sie enthalten zusätzlich übliche für Haarfestiger bekannte Farbstoffe wie beispielsweise aromatische Nitrofarbstoffe (z. B. 1,4-Diamino-2-nitrobenzol), Azofarbstoffe (z. B. C.I. Acid Brown 4), Anthrachinonfarbstoffe (z. B. C.I. Disperse Violet 4) und Triphenylmethanfarbstoffe (z. B. C.I. Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gew.-%.

Die erfindungsgemäßen Mittel zur Festigung der Frisur weisen bei gleich guter Festigung des Haares gegenüber üblichen Mitteln eine verbesserte Substantivität zum Haar, eine besonders gute Kämmbarkeit und einen guten Griff des Haares im nassen Zustand sowie einen besonders angenehmen Griff des Haares im getrockneten Zustand auf.

Wenn die erfindungsgemäßen Mittel Haarwaschmittel darstellen, liegen sie in Form wäßriger Lösungen oder Emulsionen vor und enthalten neben dem neuen Chitosanderivat zumindest ein anionisches, kationisches, nichtionisches oder amphoteres Tensid.

In diesem Haarwaschmittel liegt die Konzentration des Tensides im allgemeinen zwischen 3 und 50 Gew.-% und vorzugsweise zwischen 3 und 25 Gew.-%, bezogen auf das Gesamtgewicht des Mittels, wobei der pH-Wert im allgemeinen zwischen 3 und 9 und vorzugsweise zwischen 4 und 7 liegt.

Die erfindungsgemäßen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Copra-Fettsäuren, Lauryl- oder Ölsäuremono- oder -diethanolamid, die zweckmäßigerweise in Mengen von 1 bis 10 und vorzugsweise 1 bis 3 Gew.-%, bezogen auf das Gesamtgewicht des Mittels Verwendung finden, angeführt werden.

Unter den Verdickern können insbesondere die Acrylpolymere und die Cellulosederivate wie Carboxymethylcellulose, Hydroxypropylmethylcellulose, Hydroxyethylcellulose angeführt werden. Die Verdicker liegen im allgemeinen in einem Anteil von 0,1 bis 5 Gew.-% vor.

Unter den Tensiden oder oberflächenaktiven Agentien, die in Kombination mit den neuen quaternären Chitosanderivaten verwendet werden, können beispielsweise die folgenden genannt werden:

a) die anionischen oberflächenaktiven Agentien, wie beispielsweise die Alkali-, Erdkali-, Ammonium- oder Alkanolaminsalze von Alkansulfonaten, Alkylsulfaten und Alkylethersulfaten, die $C_{12}$-$C_{18}$-Alkyl- und insbesondere $C_{12}$-$C_{14}$-Alkyl-Sulfatnatriumsalze oder Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agentien, wie beispielsweise oxethylierte Fettalkohole mit 12 bis 18 Kohlenstoffatomen, z. B. mit bis zu 40 Mol Ethylenoxid pro Mol Fettalkohol, oxethylierter Laurin-, Tetradecyl-, Cetyl-, Olein-, Palmitin- und Stearinalkohol, allein oder im Gemisch, die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül sowie Fettsäurealkanolamide;

c) die kationischen oberflächenaktiven Agentien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Aklylpyridiniumsalze, beispielsweise Cetylpyridinumchlorid, die Alkylamidethyltrimethylammoniumethersulfate, Imidazolinderivate, Verbindungen mit kationischem Charakter wie Aminoxide, beispielsweise Aklyldimethylaminoxide oder Alkylaminoethyldimethylaminoxide;

d) die amphoteren oder zwitterionischen oberflächenaktiven Agentien wie beispielsweise die Carboxylderivate von Imidazol, die N-Alkylbetaine, die N-Alkylsulfobetaine, die N-Alkylaminobetaine, die N-Alkylaminopropionate, die Alkyldimethylammoniumacetate bzw. die $C_{12}$-$C_{18}$-Alkyldimethylcarboxymethylammoniumsalze.

Die erfindungsgemäßen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder Suspensionen vor und enthalten zusätzlich zu den neuen Chitosanderivaten der Formel I kationische, nichtionogene, amphotere oder anionische Emulgatoren sowie als Bestandteil der Ölphase z. B. Fettalkohole, Fettsäureester- oder amide, weiterhin Parfümöle, Vaseline, Wollfettalkohol oder feste oder flüssige Paraffine.

Wenn die erfindungsgemäßen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise aus der Gruppe der aromatischen Diamine bzw. Aminophenole. Weiterhin können diese Mittel gegebenenfalls Alkalisierungsmittel, Antioxidantien sowie weitere für solche Mittel übliche kosmetische Zusätze und Hilfsstoffe enthalten.

Die erfindungsgemäßen Mittel können auch Dauerverformungsmittel oder Fixiermittel für Haare darstellen. Sie enthalten dann zusätzlich zu den genannten Chitosanderivaten der Formel I Reduktionsmittel, wie zum Beispiel Thioglykolsäure, Thiomilchsäure und Ammoniumsulfit bzw. Oxidationsmittel, wie z. B. Wasserstoffperoxid oder Natriumbromat sowie gegebenenfalls Alkalisierungsagentien bzw. Peroxidstabilisatoren, z. B. Phosphorsäure, ferner andere kosmetische Hilfsstoffe und Zusatzstoffe wie beispielsweise Parfümöle, Riechstoffe, Pflegestoffe und Farbstoffe.

Wie bereits erwähnt wurde, können die erfindungsgemäßen kosmetischen Mittel auch zur Behandlung der Haut verwendet werden.

In der Tat erleichtern diese kosmetischen Mittel die Befeuchtung der Haut und verhindern das Austrocknen. Diese Mittel verleihen der Haut weiterhin eine hervorragende Weichheit im Griff.

Die erfindungsgemäßen kosmetischen Mittel liegen hierzu vorzugsweise in Form von Cremes, Gelen, Emulsionen oder wäßrigen, alkoholischen oder wäßrig-alkoholischen Lösungen vor, die das Chitosanderivat

der Formel 1 in einer Konzentration von 0,1 bis 10 Gew.-% und vorzugsweise von 0,2 bis 6 Gew.-% enthalten.

Die im allgemeinen in diesen Kosmetikzubereitungen enthaltenen Hilfsstoffe sind beispielsweise Duftstoffe, Farbstoffe, Konservierungsmittel, Verdickungsmittel, Sequestriermittel, Emulgiermittel, Sonnenschutzfilter etc..

Diese Zubereitungen für die Hautpflege liegen insbesondere in Form von Cremes oder Lotionen zur Pflege der Hände oder des Gesichts oder in Form von Sonnenschutzcremes, gefärbten Cremes, Abschminkmilchprodukten, Schaumbad- und Duschbad-Präparaten oder auch in Form von Deodorierzubereitungen vor.

Diese Zubereitungen werden unter Anwendung klassischer Verfahrensweisen hergestellt.

Beispielsweise kann man zur Bildung einer Creme eine wäßrige Phase, die das erfindungsgemäße Chitosanderivat und gegebenenfalls andere Bestandteile oder Hilfsstoffe gelöst enthält, und eine ölige Phase emulgieren.

Für die ölige Phase kann man verschiedenartige Verbindungen verwenden, beispielsweise Paraffinöl, Vaselinöl, Süßmandelöl, Avocadoöl, Olivenöl, Fettsäureester, wie Glycerylmonostearat, Ethylpalmitat oder Isopropylpalmitat oder Alkylmyristate, wie Propylmyristat, Butylmyristat oder Cetylmyristat. Man kann sie auch mit Fettsäurealkoholen, wie Cetylalkohol oder Wachsen, beispielsweise Bienenwachs, versetzen.

Die Chitosanderivate der Formel I können in den Kosmetikzubereitungen für die Hautpflege entweder als Hilfsstoff oder als Hauptwirkstoff enthalten sein.

Die in den erfindungsgemäßen kosmetischen Mitteln enthaltenen neuen Chitosan-Derivate leiten sich von Chitosan ab, einem Material, das durch Entacetylierung von Chitin, einem natürlich vorkommenden Acetylglucosamin, erhalten wird.

Das Chitosan ist im neutralen und alkalischen Medium unlöslich, es bildet jedoch auf Grund seiner chemischen Natur im sauren Medium mit organischen und anorganischen Säuren Salze, die beispielsweise in der Papier- und Textilindustrie als Additive Verwendung finden sowie weiterhin als Koagulanzien für Suspensionen, als Chelatbildner für Schwermetallionen sowie in der Medizin und in der Kosmetik benutzt werden (siehe in diesem Zusammenhang die Veröffentlichung von Muzarelli: "Chitin", Pergamon Press, 1977).

Es sind auch bereits einige wasserlösliche Chitosanderivate bekannt, beispielsweise Carboxymethylchitosan, Sulfoethylchitosan (siehe Nud'ga Plisko und Danilov, Zhur. Prikl. Khim. 47, (1974) 872-875). Diese wasserlöslichen Chitosanderivate sind jedoch in ihrem ionischen Charakter verändert oder aber physiologisch bedenklich (Epichlorhydrinchitosan, Veröffentlichung von Noguchi, Arato und Komai; Kogyo Kagaku Zasshi 72, (1969) 796-799 und japanische Patentanmeldung Nr. 46 - 39, 322, H. Haga).

Diese vorerwähnten polymeren Verbindungen erfordern außerdem für ihre technische Herstellung relativ aufwendige Verfahren.

Weiterhin berichtet unsere eigene EP-A-0 115 574 von wasserlöslichen N-substituierten quaternären Chitosanderivaten, die durch Umsetzung von Chitosan mit Glycidyltrialkylammoniumhalogeniden und Glycidol (2,3-Epoxy-1-propanol) in Gegenwart eines geeigneten Lösungsmittels erhalten werden. Die rasche Hydrolyse des Glycidols in Gegenwart von Wasser, sein hoher Preis und die Tatsache, daß Glycidol nicht großtechnisch hergestellt wird und somit nicht in beliebiger Menge verfügbar ist, verteuern jedoch das Verfahren zur Herstellung dieser Chitosanderivate.

Es wurde nunmehr gefunden, daß sich durch Umsetzung von Chitosan mit einem Glycidyltrialkylammoniumhalogenid (Oxyranmethanammonium-N,N,N-trialkylhalogenid) der Formel

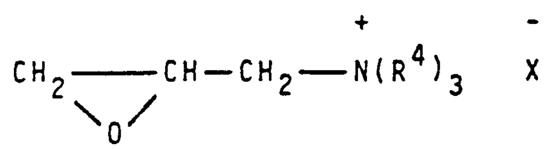

($R^4$ = $C_1$- bis $C_4$-Alkyl; X = Cl, Br, J oder $CH_3SO_4$) sowie zusätzlich mit Propylenoxid in einfacher Weise quaternäre Chitosanderivate mit hoher Substantivität, unter anderem zu Haarkeratin, herstellen lassen.

Die neuen quaternären makromolekularen, vom Chitosan abgeleiteten polymeren Verbindungen sind gekennzeichnet durch die allgemeine Formel I

$$HO[C_6H_{11-m}NO_4(R^1)_m(R^2)_n(R^3)_p]_pH, \qquad (I)$$

wobei m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

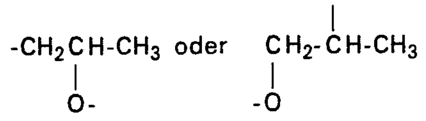

darstellt und
$R^3$ ein zweiwertiger Rest

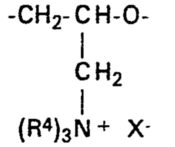

oder

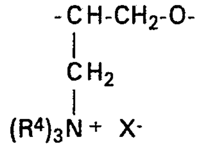

mit $R^4$ = $C_1$- bis $C_4$-Alkyl und X = Cl, Br, J oder $CH_3SO_4$ ist, wobei der Ausdruck in eckigen Klammern sich wiederholende substituierte Glucosamin-Monomereinheiten darstellen soll.

Die neuen, quaternären Stickstoff enthaltenden Chitosanderivate werden erfindungsgemäß hergestellt, indem man ein Chitosan, bestehend aus zu 50 - 100 % entacetyliertem Chitin, in Gegenwart eines Lösungsmittels mit einem Glycidyltrialkylammoniumhalogenid und Propylenoxid in geeignetem Verhältnis zur Umsetzung bringt. Bei der Umsetzung kann auch auf ein Lösungsmittel verzichtet werden, wobei dann ein Überschuß an Propylenoxid als Lösungsmittel dient.

Geeignete Glycidyltrialkylammoniumhalogenide sind zum Beispiel Glycidyltrimethylammoniumchlorid und Glycidyltriethylammoniumchlorid. Die den quaternären Stickstoff enthaltenden Epoxide können aber auch in situ durch Umsetzung der entsprechenden Chlorverbindung mit basischen Katalysatoren (beispielsweise Umsetzung von 1-Chlor-3-trimethylammonium-propanol-2-chlorid mit Natronlauge) vor oder während der Umsetzung mit dem Chitosan erzeugt werden.

Die Umsetzung des Chitosans mit Propylenoxid und dem quaternären Epoxyd kann in einfacher Weise durch gleichzeitige Umsetzung mit beiden Komponenten erfolgen, es ist jedoch auch möglich, die Umsetzung in 2 Stufen durchzuführen. So kann zunächst das Chitosan mit dem Propylenoxid und das erhaltene Produkt mit dem quaternären Epoxid oder das Chitosan zunächst mit dem quaternären Epoxid und sodann mit Propylenoxid umgesetzt werden.

Hierzu wird zweckmäßig das Chitosan in feingepulverter Form eingesetzt. Die Umsetzung selbst kann bei Temperaturen zwischen 10 und 100°C, vorzugsweise zwischen 50 und 80°C, erfolgen, und diese Temperatur wird zweckmäßig unter Rühren 2 bis 100 h beibehalten. Die Reaktion kann mit oder ohne saure oder basische Katalysatoren in Gegenwart von Lösungsmitteln oder, bei Verwendung eines Überschusses an Propylenoxid auch ohne ein besonderes Lösungsmittel erfolgen.

Vorzugsweise wird die Reaktion jedoch in Gegenwart von Wasser durchgeführt. Es hat sich dabei als vorteilhaft erwiesen, ohne zusätzliche Katalysatoren zu arbeiten, wobei das Verhältnis von Chitosan zu Wasser zwischen 1 : 0,05 und 1 : 100 und das Verhältnis von Chitosan zur Gesamtmenge aus dem Propylenoxid und dem Glycidyltrialkylammoniumhalogenid (bezogen auf die Mole von substituierbaren Aminogruppen am Chitosan) zwischen 1 : 0,5 und 1 : 30, vorzugsweise zwischen 1 : 1 und 1 : 10, liegt. Das Verhältnis zwischen dem Propylenoxid und dem Glycidyltrialkylammoniumhalogenid soll bei der Umsetzung je nach gewünschtem Substitutionsgrad, zweckmäßigerweise zwischen 1 : 100 und 100 : 1, vorzugsweise zwischen 1 : 10 und 10 : 1, liegen.

Obwohl die Durchführung der Reaktion in Gegenwart von Wasser bevorzugt ist, kann sie auch unter Verwendung anderer Lösungsmittel, in denen mindestens eines der Reaktionsprodukte löslich ist, durchgeführt werden. Beispiele für solche Lösungsmittel sind Alkohole wie Ethanol, Methanol, Glykol und Glycerin sowie Ketone, beispielsweise Methylethylketon und Aceton.

Gemäß einer anderen Ausführungsform des erfindungsgemäßen Verfahrens zur Herstellung der Chitosanderivate der Formel I werden bei der Umsetzung zusätzlich organische oder anorganische Säuren oder Basen als Katalysatoren zugesetzt.

Für einen Einsatz als Katalysatoren geeignete Säuren sind zum Beispiel Salzsäure, Milchsäure und Ameisensäure. Beispiele für geeignete Basen sind Trialkylamine wie beispielsweise Trimethylamin, Triethylamin oder Trialkylolamine sowie Alkalihydroxide und Erdalkalihydroxide. Es ist grundsätzlich auch möglich, von wasserlöslichen Salzen des Chitosans, beispielsweise Chitosanlaktat, Chitosanacetat oder Chitosanhydrochlorid auszugehen. Hierbei kann jedoch als Nebenprodukt eine größere Menge der Glycerinester der verwendeten Säure entstehen, so daß die Reinigung der entstandenen Reaktionsprodukte erschwert wird.

Die Aufarbeitung der Reaktionsgemische kann beispielsweise in der Weise erfolgen, daß man das Lösungsmittel und gegebenenfalls überschüssiges Propylenoxid im vakuum aus der Reaktionsmischung abdestilliert.

Bei der Herstellung von wasserlöslichen quaternären Chitosanderivaten kann das Reaktionsprodukt in bevorzugter Weise in einem Überschuß an Wasser gelöst und durch Filtration oder Zentrifugieren vom nichtlöslichen Reaktionsrückstand abgetrennt werden. Als weitere Schritte zur Reinigung der Reaktionsprodukte können die wäßrigen Lösungen dialysiert werden und/oder gegebenenfalls nach Einengen der wäßrigen Lösungen durch Ausfällen in Aceton, Alkoholen oder anderen organischen Lösungsmitteln isoliert werden.

Gemäß einer besonders vorteilhaften Ausführungsform des erfindungsgemäßen Herstellungsverfahrens wird als Ausgangsmaterial durch Umfällen und Tiefgefrieren strukturell modifiziertes Chitosan eingesetzt. Mit einem solchen Ausgangsmaterial verläuft die Reaktion in besonders vorteilhafter Weise und in besonders guter Ausbeute.

Anhand der nachfolgenden Beispiele wird das erfindungsgemäße Verfahren zu Herstellung der neuen quaternären Chitosanderivate näher erläutert.

**Herstellungsbeispiele**

**Umsetzungen von Chitosan mit Glycidyltrimethylammoniumchlorid und Propylenoxid**

**Beispiel 1**

25 g (0,155 mol) Chitosan mit einer Grenzviskositätszahl von ($\eta_=$ 1600 ml/g) und 76 % freies Amin, wurden mit der äquimolaren Menge Salzsäure in 5 l Wasser gelöst und anschließend durch Einstellen eines pH-Wertes von 9,5 mit Natronlauge ausgefällt.

Das ausgefällte und abgesaugte, wasserhaltige Chitosan wurde im Autoklav (2 l Rauminhalt) mit 39,5 ml Propylenoxid und 53,5 g einer 60 %-igen Glycidyltrimethylammoniumchloridlösung 6 Stunden lang bei 80 C gerührt. Dabei wurde ein Druckanstieg auf 0,7 bar registriert.

Das klar wasserlösliche Reaktionsprodukt, welches honigartige Konsistenz hatte, wurde durch Eintropfen in 15 l Aceton unter starkem Rühren ausgefällt, abgesaugt und anschließend bei 50°C im vakuum getrocknet.

Es wurden 29,8 g ouaternäres Chitosanderivat erhalten.

**Kenndaten des quaternären Chitosanderivates**

| | |
|---|---|
| Grenzviskositätszahl: | 610 ml/g |
| Titrierbarer Stickstoff: | 3,37 mmol/g |
| Chloridbestimmung: | 0,95 mmol/g |
| Kolloid-Titration: | 0,95 mmol/g |

Substitutionsgrad daraus berechnet: q = 0,28; n = 1,52

Zur Berechnung des Substitutionsgrades wird zunächst die Molmenge an titrierbarem Stickstoff $N_t$ mmol/g durch nichtwäßrige Titration mit Perchlorsäure bestimmt. Das mittlere Molekulargewicht einer substituierten Chitosaneinheit berechnet sich daraus wie folgt:

$$\bar{M}\left[\frac{g}{Mol}\right] = \frac{1000}{N_t\left[\frac{mmol}{g}\right]}$$

Weiterhin gilt die Gleichung

$\bar{M} = 161 + (q \cdot 152) + (n \cdot 57) + (m \cdot 42)$

| | |
|---|---|
| - Molekulargewicht einer Chitosaneinheit = | 161 [g/mol] |
| - Molekulargewicht einer quaternären Gruppe $R_3$ aus Formel I ($R^4$ = $CH_3$; X = Cl) = | 152 [g/mol] |
| - Molekulargewicht einer Propylenoxid-Gruppe $R^2$ aus Formel I = | 57 [g/mol] |
| - Molekulargewicht einer $CO\text{-}CH_2$-Gruppe = | 42 [g/mol] |

q = Substitutionsgrad, kationische Gruppen $R^3$

n = Substitutionsgrad Propyl-Gruppen $R^2$

m = Substitutionsgrad des verwendeten Chitosans mit Acetyl-Gruppe (bei 60 - 96 % freien Aminogruppen beträgt der Substitutionsgrad 0,04 - 0,4)

Zur Berechnung des Substitutionsgrades mit kationischen Gruppen $R^3$ wird die Kolloid-Titration der kationischen Gruppen mit Polyvinylsulfat-Kaliumsalzlösung herangezogen. Es gilt

$$q = \frac{[kolloid] \cdot \bar{M}}{1000}$$

Daraus läßt sich der Substitutionsgrad mit Propylgruppen wie folgt berechnen.

$$n = \frac{\bar{M} - 161 - 152 \cdot q - 42 \cdot m}{57}$$

**Beispiel 2**

25 g Chitosan mit den gleichen Kenndaten wie in Beispiel 1 wurden wiederum in Salzsäure gelöst und durch Zugabe von Natronlauge wieder ausgefällt. Anschließend wurde die Reaktion wie in Beispiel 1 im Autoklaven durchgeführt.

Dazu wurden 45,6 ml Propylenoxid und 31,5 g einer 60 %-igen Glycidyltrimethylammoniumchloridlösung zugegeben. Nach Ende der Reaktionszeit wurde das nicht vollkommen klar wasserlösliche Produkt auf etwa 4 l verdünnt und durch Plattenfilter klar filtriert. Danach wurde die wäßrige Lösung im Rotationsverdampfer auf etwa 2 l aufkonzentriert.

Das quaternäre Chitosanderivat wurde anschließend durch Eintropfen der Lösung in 20 l Aceton ausgefällt, abgesaugt und sodann bei 50°C im Vakuum getrocknet. Es wurden 25,3 g des quaternären Chitosanderivates erhalten.

**Kenndaten des quaternären Chitosanderivats**

| | |
|---|---|
| Grenzviskositätszahl: | 563 ml/g |
| Titrierbarer Stickstoff: | 2,6 mmol/g |
| Chloridbestimmung: | 2,55 mmol/g |
| Kolloid-Titration: | 3,02 mmol/g |

Substitutionsgrad daraus berechnet: q = 1,16; n = 0,72

**Beispiel 3**

100 g (0,621 mol) Chitosan mit einer Grenzviskositätszahl von $\eta$ = 140 ml/g (ermittelt im DIN-Ubbelohde Viskosimeter mit 0,2 m Essigsäure und 0,1 m Natriumacetat als Lösungsmittel) und einem freien Amin-Gehalt von 86 % wurden wie in Beispiel 1 beschrieben ausgefällt und mit den Epoxiden im Autoklaven umgesetzt.

Dazu wurden 182,4 g Propylenoxid und 126 g einer 60 %-igen wäßrigen Lösung von Glycidyltrimethylammoniumchlorid zugegeben.

Nach Ende der Reaktionszeit (6 h bei 80°C) wurde die wäßrige Lösung des Reaktionsproduktes in 10 l Aceton ausgefällt, abgesaugt und anschließend bei 50°C im vakuum getrocknet.

Es wurden 124,6 g des quaternären Chitosanderivates erhalten.

**Kenndaten des quaternären Chitosanderivates**

| | |
|---|---|
| Grenzviskositätszahl: | 35 ml/g |
| Titrierbarer Stickstoff: | 3,24 mmol/g |
| Chloridbestimmung: | 1,82 mmol/g |
| Kolloid-Titration: | 1,64 mmol/g |

Substitutionsgrad daraus berechnet: q = 0,51; n = 1,14

**Beispiele für kosmetische Mittel**

**Beispiel 4**

**Haarfestiger**

| | |
|---|---|
| 0,6 g | quaternäres Chitosanderivat nach Beispiel 3 ($\eta$ = 35 ml/g, Substitutionsgrad = kationische Gruppen 0,51; Propyl-Gruppen 1,14) |
| 73,8 g | Wasser |
| 25,0 g | Isopropanol |
| 0,4 g | 10 %-ige Ameisensäure |
| 0,2 g | Parfümöl |
| 100,0 g | |

20 ml dieser Lösung wurden auf gewaschenem, handtuchtrockenem Haar verteilt, das Haar in üblicher Weise zur Frisur eingelegt und getrocknet. Bei guter Festigungswirkung zeigte das Haar, im Vergleich zu einem Haarfestiger auf der Basis von Chitosan/Ameisensäure, einen angenehmeren und weicheren Griff.

**Beispiel 5**

**Tönungsfestiger**

| | |
|---|---|
| 1,00 g | quaternäres Chitosanderivat nach Beispiel 2 (η = 563 ml/g, Substitutionsgrad = kationische Gruppen 1,16; Propyl-Gruppen 0,72) |
| 1,00 g | Milchsäure |
| 0,10 g | Cetyltrimethylammoniumchlorid, 50 %-ige wäßrige Lösung |
| 0,05 g | Acid Brown 4 (C.I. 41 805) |
| 97,85 g | Wasser |
| 100,00 g | |

20 ml dieser Lösung wurden auf dem gewaschenen, handtuchtrockenen Haar verteilt und das Haar in üblicher Weise eingelegt und getrocknet. Das Haar zeigte anschließend eine leichte Rot-Braunfärbung.

**Beispiel 6**

**Tönungsfestiger**

| | |
|---|---|
| 0,60 g | quaternäres Chitosanderivat nach Beispiel 2 (η= 563 ml/g, Substitutionsgrad = kationische Gruppen 1,16; Propyl-Gruppen 0,72) |
| 0,15 g | 1,4-Di(β -hydroxyethylamino)-2-nitro-5-chlorbenzol |
| 25,00 g | Ethanol |
| 74,25 g | Wasser |
| 100,00 g | |

20 ml dieser Lösung wurden auf die gewaschenen, handtuchtrockenen Haare gegeben, sodann das Haar eingelegt und getrocknet. Die Haare waren rot-violett gefärbt und gefestigt.

**Beispiel 7**

**Anionisches Haarwaschmittel**

| | |
|---|---|
| 1,00 g | quaternäres Chitosanderivat nach Beispiel 1 (η= 610 ml/g, Substitutionsgrad = kationische Gruppen 0,28; Propyl-Gruppen 1,52) |
| 40,00 g | Laurylalkoholdiglykolethersulfat-Natriumsalz, 28 %-ige wäßrige Lösung |
| 4,00 g | Natriumchlorid |
| 0,05 g | Farbstoff |
| 54,85 g | Wasser |
| 0,10 g | Formaldehyd, 25 %-ige wäßrige Lösung |
| 100,00 g | |

Es wurde ein klares Shampoo erhalten. Das damit gewaschene Haar war hinsichtlich Griff, Glanz und Kämmbarkeit ausgezeichnet konditioniert. Infolge der Verträglichkeit des quaternären Chitosanderivates mit Alkylethersulfat kann das vorstehend aufgeführte Shampoo erstellt werden, mit dem eine gleichzeitige Reinigung und Pflege des Haares möglich ist.

**Beispiel 8**

**Amphoteres, tönendes Haarwaschmittel**

| | |
|---|---|
| 2,00 g | quaternäres Chitosanderivat nach Beispiel 1 (η = 610 ml/g, Substitutionsgrad = kationische Gruppen 0,28; Propyl-Gruppen 1,52) |
| 40,00 g | Dimethyl-carboxymethylen-propylenamido-stearatbetain, 35 %-ige wäßrige Lösung |
| 5,06 g | Ameisensäure, 10 %-ig |
| 3,50 g | Kokosfettsäurediethanolamid |
| 1,00 g | Pikraminsäure (C.I. 76 540), 1 %-ige wäßrige Lösung) |
| 48,44 g | Wasser, vollentsalzt |
| 100,00 g | |

Das Haar wurde mit etwa 20 g des obigen Mittels einshampooniert. Nach einer Einwirkungszeit von 5 bis 10 Minuten spülte man mit Wasser aus. Das Haar war gelborange getönt und ausgezeichnet konditioniert, besonders hinsichtlich Griff und Kämmbarkeit.

**Beispiel 9**

**Haarkurmittel, kationisch**

| | |
|---|---|
| 0,30 g | quaternäres Chitosanderivat nach Beispiel 3 (η = 35 ml/g, Substitutionsgrad = kationische Gruppen 0,51; Propyl-Gruppen 1,14) |
| 4,00 g | Cetylstearylalkohol |
| 1,48 g | Milchsäure, 10 %-ig |
| 2,50 g | Kokos(pentaethoxy)methylammoniumchlorid |
| 1,00 g | Sorbitanmonopalmitat mit 20 Mol Ethylenoxid |
| 90,72 g | Wasser, vollentsalzt |
| 100,00 g | |

**Beispiel 10**

**Haarkurmittel, gelförmig**

| | |
|---|---|
| 2,10 g | quaternäres Chitosanderivat nach Beispiel 1 (η = 610 ml/g, Substitutionsgrad = kationische Gruppen 0,28 Propyl-Gruppen 1,52) |
| 0,60 g | Hydroxypropylmethylcellulose |
| 0,50 g | Laurylpyridiniumchlorid |
| 96,80 g | Wasser, vollentsalzt |
| 100,00 g | (auf pH 5,0 mit 10 %-iger Ameisensäure eingestellt) |

Jeweils 35 g der Haarkurmittel nach Beispiel 9 bzw. 10 wurden im gewaschenen Haar verteilt und nach einer Einwirkungszeit von 3 bis 5 Minuten mit Wasser wieder ausgespült; als Ergebnis wurde ein ausgezeichneter Griff, Glanz sowie Kämmbarkeit des Haares festgestellt.

**Beispiel 11**

**Hautcreme**

| | |
|---|---|
| 0,30 g | quaternäres Chitosanderivat nach Beispiel 3 (η = 35 ml/g, Substitutionsgrad = kationische Gruppen 0,51; Propyl-Gruppen 1,14) |
| 3,00 g | Stearylalkohol |
| 1,00 g | Wollfettalkohol (Adeps Lanae) |
| 1,00 g | Vaseline |
| 0,76 g | Milchsäure, 10 %-ig |
| 1,00 g | Natriumcetylstearylsulfat |
| 92,94 g | Wasser, vollentsalzt |
| 100,00 g | |

**Beispiel 12**

**Haartönungsmittel**

| | |
|---|---|
| 0,50 g | quaternäres Chitosanderivat nach Beispiel 2 (η = 563 ml/g, Substitutionsgrad = kationische Gruppen 1,16; Propyl-Gruppen 0,72) |
| 12,00 g | Cetylstearylalkohol |
| 0,10 g | Parahydroxybenzoesäureethylester |
| 6,00 g | Laurylalkohol-diglycolethersulfat-Natriumsalz (28 %-ige wäßrige Lösung) |
| 0,50 g | Parfümöl |
| 79,31 g | Wasser |
| 0,50 g | 1-Hydroxy-2-amino-4-nitrobenzol (C.I. 76 530) |
| 0,85 g | 1,4-Diamino-2-nitrobenzol (C.I. 76 070) |
| 0,24 g | Natriumhydroxyd |
| 100,00 g | |

Ungefähr 30 bis 40 g wurden in dem gewaschenen Haar verteilt und nach einer Einwirkungszeit von 20

Minuten ausgespült. Das Haar war rötlich gefärbt und wies eine gute Kämmbarkeit und einen angenehmen Griff auf.

**Beispiel 13**

**Oxidationshaarfärbemittel**

| | |
|---|---|
| 0,50 g | quaternäres Chitosanderivat nach Beispiel 2 ($\eta$ = 563 ml/g, Substitutionsgrad = kationische Gruppen 1,16; Propyl-Gruppen 0,72) |
| 0,08 g | 3,5-Diamino-2,6-dimethoxypyridin-dihydrochlorid |
| 0,30 g | 1,4-Diaminobenzol |
| 0,25 g | Resorcin |
| 0,30 g | Natriumsulfit |
| 3,50 g | Laurylalkohol-diglycolethersulfat-Natriumsalz (28 %-ige wäßrige Lösung) |
| 15,00 g | Cetylalkohol |
| 3,00 g | Ammoniak |
| 77,07 g | Wasser |
| 100,00 g | |

50 g dieses Haarfärbemittels wurden mit 50 ml 6 %-iger Wasserstoffperoxidlösung gemischt und auf weißes Haar aufgetragen. Nach 30 Minuten wurde das Haar mit Wasser ausgespült und getrocknet. Das Haar hatte eine natürlich wirkende matt-blonde Färbung sowie einen natürlichen angenehmen Griff erhalten.

**Beispiel 14**

**Dauerwellmittel**

| | |
|---|---|
| 0,50 g | quaternäres Chitosanderivat nach Beispiel 3 ($\eta$ = 35 ml/g, Substitutionsgrad = kationische Gruppen 0,51; Propyl-Gruppen 1,14) |
| 10,00 g | Thioglykolsäure |
| 8,00 g | Ammoniak, 25 %-ig |
| 6,10 g | Ammoniumhydrogencarbonat |
| 75,40 g | Wasser |
| 100,00 g | |

Zur Anwendung trug man dieses Dauerwellmittel auf das gewickelte handtuchtrockene Haar gleichmäßig auf und ließ es etwa 20 Minuten einwirken; danach wurde das Haar mit Wasser ausgespült und in bekannter Weise oxidativ behandelt. Es wurde eine gutes Wellergebnis erhalten, die Haare fühlten sich natürlich und weich an.

**Beispiel 15**

**Haarfestiger alkoholfrei**

| | |
|---|---|
| 0,70 g | quaternäres Chitosanderivat nach Beispiel 3 ($\eta$ = 35 ml/g, Substitutionsgrad = kationische Gruppen 0,51; Propyl-Gruppen 1,14) |
| 1,50 g | Ameisensäure 10 %-ig |
| 0,80 g | Parfüm |
| 0,10 g | Chloracetamid (Konservierungsmittel) |
| 96,90 g | Wasser, vollensalzt |
| 100,00 g | |

20 ml dieser Lösung wurden auf die gewaschenen handtuchtrockenen Haare gegeben, sodann das Haar eingelegt und getrocknet. Bei guter Festigungswirkung zeigte das Haar einen angenehmen weichen Griff.

**Patentansprüche**

1. Kosmetisches Mittel zur Behandlung von Haaren oder der Haut, dadurch gekennzeichnet, daß es in einer geeigneten Kosmetikgrundlage eine quaternäre makromolekulare, vom Chitosan abgeleitete polymere

Verbindung der allgemeinen Formel I

$$HO[C_6H_{11-m}NO_4(R^1)_m(R^2)_n(R^3)_q]_pH, \quad (I)$$

wobei
m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

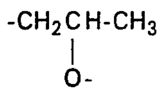 oder 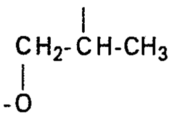

darstellt und $R^3$ ein zweiwertiger Rest

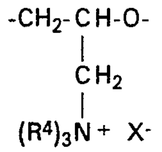 oder 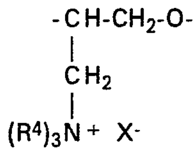

mit $R^4$ = $C_1$- bis $C_4$-Alkyl und X = Cl, Br, J oder $CH_3SO_4$ ist, enthält.

2. Mittel nach Anspruch 1, dadurch gekennzeichnet, daß n in der Formel I einen Zahlenwert von 0,72 bis 1,52 bedeutet.

3. Mittel nach Anspruch 1 und 2, dadurch gekennzeichnet, daß q in der Formel I einen Zahlenwert von 0,28 bis 1,16 bedeutet.

4. Mittel nach Anspruch 1 bis 3, dadurch gekennzeichnet, daß es die polymere Verbindung der Formel I in einer Menge von 0,05 bis 10,0 Gew.-% enthält.

5. Mittel nach Anspruch 1 bis 4, dadurch gekennzeichnet, das es als Kosmetikgrundlage eine wäßrige, alkoholische oder wäßrig-alkoholische Lösung, eine Creme, ein Gel oder eine Emulsion enthält.

6. Mittel nach Anspruch 1 bis 5, dadurch gekennzeichnet, daß es als Kosmetikgrundlage eine wäßrige oder wäßrig-alkoholische Lösung eines niedermolekularen Alkohols, wie Ethanol oder Isopropanol, umfaßt und einen pH-Wert zwischen 6 und 8 aufweist.

7. Mittel nach Anspruch 1 bis 5, dadurch gekennzeichnet, daß es zusätzlich ein kationisches, nicht-ionisches, amphoteres oder anionisches Tensid enthält und in Form eines Haarwaschmittels vorliegt.

8. Mittel nach Anspruch 7, dadurch gekennzeichnet, daß das anionische Tensid ein Alkylethersulfat ist.

9. Mittel nach Anspruch 7 und 8, dadurch gekennzeichnet, daß es das Tensid in einer Konzentration zwischen 3 und 25 Gew. % enthält und einen pH-Wert zwischen 4 und 7 aufweist.

10. Mittel nach Anspruch 1 bis 6, dadurch gekennzeichnet, daß es als Kosmetikgrundlage eine alkoholische oder wäßrig-alkoholische Lösung enthält, die mit einem unter Druck verflüssigten Treibgas vermischt ist, in einem Druckbehälter abgefüllt ist und in Form eines Aerosolhaarsprays vorliegt.

11. Mittel nach Anspruch 1 bis 6, dadurch gekennzeichnet, daß die Kosmetikgrundlage eine wäßrige, alkoholische oder wäßrig-alkoholische Lösung, eine Creme, ein Gel oder eine Emulsion ist, in der das Chitosanderivat der Formel I in einer Konzentration zwischen 0,1 und 10 Gew.-% enthalten ist und es in Form eines Hautbehandlungsmittels vorliegt.

12. Mittel nach Anspruch 1 bis 10, dadurch gekennzeichnet, daß es zusätzlich ein bekanntes filmbildendes synthetisches oder natürliches kosmetisches Polymer enthält.

13. Mittel nach Anspruch 1 bis 5, dadurch gekennzeichnet, daß die Kosmetikgrundlage eine wäßrige, alkoholische oder wäßrig-alkoholische Lösung oder eine Emulsion ist, welche zusätzlich einen Farbstoff enthält und in Form eines Farbfestigers oder Tönungsfestigers vorliegt.

14. Quaternäre makromolekulare, vom Chitosan abgeleitete Polymere Verbindungen der allgemeinen Formel I

$$HO[C_6H_{11-m}NO_4(R^1)_m(R^2)_n(R^3)_q]_pH, \quad (I)$$

wobei
m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,

$R^2$ einen zweiwertigen Rest

```
                         |
-CH2CH-CH3  oder   CH2-CH-CH3
    |              |
    O-            -O
```

darstellt und $R^3$ ein zweiwertiger Rest

```
-CH2-CH-O-   oder    -CH-CH2-O-
     |                 |
     CH2               CH2
     |                 |
(R4)3N+ X-         (R4)3N+ X-
```

mit $R^4$ = $C_1$-$C_4$-Alkyl und X = Cl, Br, J oder $CH_3SO_4$ ist.

15. Verbindung gemäß Anspruch 14, dadurch gekennzeichnet, daß in der Formel I n einen Zahlenwert von 0,72 bis 1,52 bedeutet, q einen Zahlenwert von 0,28 bis 1,16 darstellt und die Grenzviskositätszahl einen Wert von 35 bis 610 ml/g aufweist.

16. Verbindung gemäß Anspruch 14, dadurch gekennzeichnet, daß in der Formel I n = 1,14 bedeutet und q = 0,51 ist.

17. Verbindung gemäß Anspruch 14, dadurch gekennzeichnet, daß in der Formel I $R^4$ = $CH_3$ und X = Cl bedeutet.

18. Verfahren zur Herstellung der Verbindungen nach Anspruch 14, dadurch gekennzeichnet, daß man ein Chitosan, bestehend aus zu 50 - 100 % entacetyliertem Chitin, mit einem Glycidyltrialkylammoniumhalogenid sowie zusätzlich mit Propylenoxid in geeignetem Verhältnis gleichzeitig oder in beliebiger Reihenfolge zur Umsetzung bringt.

19. verfahren nach Anspruch 18, dadurch gekennzeichnet, daß man das Chitosan in Gegenwart eines Lösungsmittels mit dem Glycidyltrialkylammoniumhalogenid und dem Propylenoxid vermischt und diese Mischung bei einer Temperatur zwischen 10 und 100°C mehrere Stunden mischt oder rührt.

20. Verfahren nach Anspruch 18 und 19, dadurch gekennzeichnet, daß die Umsetzung in Gegenwart einer organischen oder anorganischen Base durchgeführt wird.

21. Verfahren nach Anspruch 18 und 19, dadurch gekennzeichnet, daß die Reaktion in Gegenwart einer organischen oder anorganischen Säure durchgeführt wird.

22. Verfahren nach Anspruch 18 bis 21, dadurch gekennzeichnet, daß man als Glycidyltrialkylammoniumhalogenid Glycidyltrimethylammoniumchlorid verwendet.

23. Verfahren nach Anspruch 18 bis 22, dadurch gekennzeichnet, daß man als Ausgangsmaterial durch Umfällen und Tiefgefrieren strukturell modifiziertes Chitosan verwendet.

24. Verfahren nach Anspruch 18 bis 23 dadurch gekennzeichnet, daß man die Umsetzung in Gegenwart von Wasser durchführt.

## Claims

1. Cosmetic medium for treating hair or skin, characterised in that it contains, in a suitable cosmetic base, a quaternary macromolecular polymeric compound derived from chitosan, of the general formula

$$HO[C_6H_{11-m}NO_4(R^1)_m(R^2)_n(R^3)_q]_pH \qquad (I),$$

wherein

m has any numerical value from 0 to 0.5,
n has any numerical value from 0.01 to 6,
q has any numerical value from 0.005 to 3,
p is an integer from 10 to 50000,
$R^1$ is an acetyl residue,
$R^2$ is a bivalent residue

```
                         |
-CH2CH-CH3  oder   CH2-CH-CH3
    |              |
    O-            -O
```

and $R^3$ is a bivalent residue

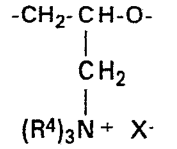
or
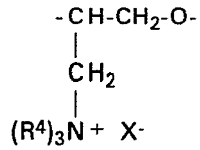

with $R^4 = C_1$- to $C_4$-alkyl and X = Cl, Br, I or $CH_3SO_4$.

2. Medium according to Claim 1, characterised in that n in formula I has a numerical value from 0.72 to 1.52.

3. Medium according to Claim 1 and 2, characterised in that q in formula I has a numerical value from 0.28 to 1.16.

4. Medium according to Claim 1 to 3, characterised in that it contains the polymeric compound of formula I in a quantity of 0.05 to 10 weight %.

5. Medium according to Claim 1 to 4, characterised in that the cosmetic base is an aqueous, alcoholic or aqueous-alcoholic solution, a cream, a gel or an emulsion.

6. Medium according to Claim 1 to 5, characterised in that it contains as cosmetic base, an aqueous or aqueous-alcoholic solution of a low molecular weight alcohol, such as ethanol or isopropanol and has a pH value between 6 and 8.

7. Medium according to Claim 1 to 5, characterised in that it additionaly contains a cationic, non-ionogenic, amphoteric or anionic surfactant and is in the form of a hair shampoo.

8. Medium according to Claim 7, characterised in that the anionic surfactant is an alkylethersulphate.

9. Medium according to Claim 7 and 8, characterised in that it contains the surfactant in a concentration of between 3 and 25 weight % and has a pH-value of between 6 an 7.

10. Medium according to Claim 1 to 6, characterised in that the cosmetic base is an alcoholic or aqueous-alcoholic solution, which is mixed with a propellant gas liquefied under pressure, charged into a pressure container and is in the form of an aerosol hair spray.

11. Medium according to Claim 1 to 6, characterised in that the cosmetic base is an aqueous, alcoholic or aqueous-alcoholic solution, a creme, a gel or an emulsion, in which the chitosan derivative of formula I is contained in a concentration of between 0.1 and 10 weight %, and which is in the form of a skin treatment composition.

12. Medium according to Claim 1 to 10, characterised in that it also contains a known film-forming synthetic or natural cosmetic polymer.

13. Medium according to Claim 1 to 5, characterised in that the cosmetic base is an aqueous, alcoholic or aqueous-alcoholic solution or an emulsion, which also contains a dye and is in the form of a colour or tone strengthener.

14. Quaternary macromolecular polymeric compound derived from chitosan of the general formula I

$$HO[C_6H_{11-m}NO_4(R^1)_m(R^2)_n(R^3)_q]_pH \qquad (I),$$

wherein
m has any numerical value from 0 to 0.5,
n has any numerical value from 0.01 to 6,
q has any numerical value from 0.005 to 3.0,
p is an integer from 10 to 50000,
$R^1$ is an acetyl residue,
$R^2$ is a bivalent residue

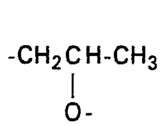
or
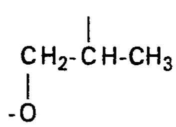

and $R^3$ is a bivalent residue

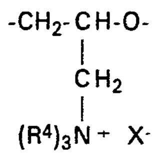
oder
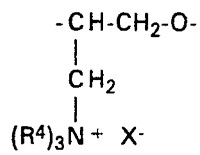

$R^4 = C_1$- to $C_4$-alkyl and X = Cl, Br, I or $CH_3SO_4$.

15. Compound according to Claim 14, characterised in that in formula I n has a numerical value between 0.72 and 1.52, q has a numerical value from 0.28 to 1.16 and the limiting viscosity number has a numerical value from 35 to 610 ml/g.

16. Compound according to Claim 14, characterised in that in formula I n is equal to 1.14 and q is equal to 0.51.

17. Compound according to Claim 14, characterised in that in formula I $R^4$ is $CH_3$ and X is Cl.

18. Process for preparing the compound according to Claim 14, characterised in that chitosan consisting of 50 to 100 % of deacetylated chitin is reacted with glycidyltrialkylammoniumhalide and propylene oxide in a suitable ratio, simultaneous or in a given sequence.

19. Process according to Claim 18, characterised in that the chitosan is mixed with the glycidyltrialkylammoniumhalide and the propylene oxide in the presence of a solvent and the resulting mixture is mixed or stirred for several hours at a temperature of between 10 and 100 °C.

20. Process according to Claim 18 and 19, characterised in that the reaction is carried out in the presence of an organic or inorganic base.

21. Process according to Claim 18 and 19, characterised in that the reaction is carried out in the presence of an organic or inorganic acid.

22. Process according to Claim 18 to 21, characterised in that the glycidyltrialkylammoniumhalide is a glycidyltrimethylammoniumchloride.

23. Process according to Claim 18 to 22, characterised in that as basic material a chitosan is used which is structurally modified by reprecipitation and deep-freezing.

24. Process according to Claim 18 to 23, characterised in that the reaction is carried out in the presence of water.

**Revendications**

1. Produit cosmétique pour le traitement des cheveux ou de la peau caractérisé en ce qu'il renferme, dans un substrat cosmétiqué approprié, un composé polymère quaternaire macromoléculaire dérivé du chitosane, répondant à la formule générale (I):

$$HO[C_6H_{11-m}NO_4(R^1)_m(R^2)_n(R^3)_q]_pH, \quad (I)$$

dans laquelle
m représente n'importe quelle valeur numérique de 0 à 0,5,
n n'importe quelle valeur numérique de 0,01 à 6,
q une valeur numérique quelconque de 0,005 à 3,0,
p un nombre entier de 10 à 50.000,
$R^1$ un reste acétyle,
$R^2$ un reste bivalent

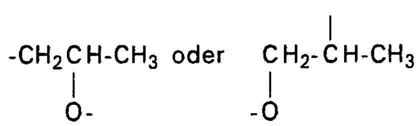

et $R^3$ un reste bivalent

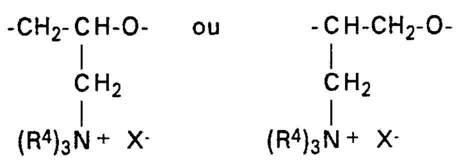

avec $R^4$ = un alkyle en $C_1$ à $C_4$ et X = Cl, Br, I ou $CH_3SO_4$.

2. Produit selon la revendication 1, caractérisé en ce que, dans la formule (I), n représente une valeur numérique de 0,72 à 1,52.

3. Produit selon les revendications 1 et 2, caractérisé en ce que, dans la formule (I), q représente une valeur numérique de 0,28 à 1,16.

4. Produit selon l'une quelconque des revendications 1 à 3, caractérisé en ce qu'il renferme le composé polymère répondant à la formule (I) dans une proportion de 0,05 à 10,0 % en poids.

5. Produit selon l'une quelconque des revendications 1 à 4, caractérisé en ce qu'il renferme, comme substrat cosmétique, une solution aqueuse, alcoolique ou hydro-alcoolique, une crème, un gel ou une émulsion.

6. Produit selon l'une quelconque des revendications 1 à 5, caractérisé en ce qu'il renferme, comme substrat cosmétique, une solution aqueuse ou hydro-alcoolique d'un alcool inférieur, comme l'éthanol ou l'isopropanol, et en ce qu'il présente une valeur de pH comprise entre 6 et 8.

7. Produit selon l'une quelconque des revendications 1 à 5, caractérisé en ce qu'il renfermé en plus un agent tensio-actif cationique, non-ionique, amphotère ou anionique, et en ce qu'il se présente sous la forme d'un produit de lavage des cheveux.

8. Produit selon la revendication 7, caractérisé en ce que l'agent tensio-actif anionique est un éther-sulfate d'alkyle.

9. Produit selon les revendications 7 et 8, caractérisé en ce qu'il renferme l'agent tensio-actif à une concentration comprise entre 3 et 25 % en poids, et en ce qu'il présente une valeur de pH comprise entre 4 et 7.

10. Produit selon les revendications 1 à 6, caractérisé en ce qu'il renferme, comme substrat cosmétique, une solution alcoolique ou hydro-alcoolique mélangée avec un gaz propulseur liquéfié sous pression, contenue dans un réservoir pressurisé et se présentant sous la forme d'un spray capillaire d'aérosol.

11. Produit selon l'une quelconque des revendications 1 à 6, caractérisé en ce que le substrat cosmétique est une solution aqueuse, alcoolique ou hydro-alcoolique, une crème, un gel ou une émulsion, dans laquelle le dérivé du chitosane répondant à la formule (I) est contenu à une concentration comprise entre 0,1 et 10 % en poids, et en ce qu'il se présente sous la forme d'un produit de traitement de la peau.

12. Produit selon les revendications 1 à 10, caractérisé en ce qu'il renferme, en plus, un polymère cosmétique filmogène synthétique ou naturel, connu.

13. Produit selon l'une quelconque des revendications 1 à 5, caractérisé en ce que le substrat cosmétique est une solution aqueuse, alcoolique ou hydro-alcoolique, ou une émulsion, qui renferme, en plus, un colorant et se présente sous la forme d'un fixateur de couleur ou d'un fixateur de nuance.

14. Composés polymères quaternaires macromoléculaires dérivant du chitosane, répondant à la formule générale (I)

$$HO[C_6H_{11-m}NO_4(R^1)_m(R^2)_n(R^3)_q]_pH, \qquad (I)$$

dans laquelle

m représente n'importe quelle valeur numérique de 0 à 0,5,
n n'importe quelle valeur numérique de 0,01 à 6,
q une valeur numérique quelconque de 0,005 à 3,0,
p un nombre entier de 10 à 50.000,
$R^1$ un reste acétyle,
$R^2$ un reste bivalent

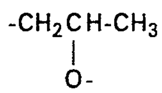 ou 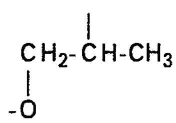

et $R^3$ un reste bivalent

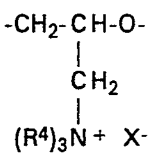 ou 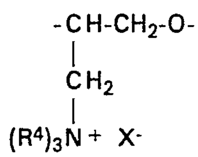

avec $R^4$ = alkyle en $C_1$ à $C_4$ et X = Cl, Br, I ou $CH_3SO_4$.

15. Composé selon la revendication 14, caractérisé en ce que, dans la formule (I), n représente une valeur numerique de 0,72 à 1,52, q une valeur numérique de 0,28 à 1,16, et en ce que l'indice de viscosité limite présente une valeur de 35 à 610 ml/g.

16. Compose selon la revendication 14, caracterisé en ce que, dans la formule (I), n = 1,14 et q = 0,51.

17. Composé selon la revendication 14, caractérisé en ce que, dans la formule (I), $R^4$ = $CH_3$ et X = Cl.

18. Procédé de préparation des composés selon la revendication 14, caractérisé en ce qu'on fait réagir un chitosane comprenant de la chitine désacétylée à 50 à 100 % sur un halogénure de glycidyltrialkylammonium, ainsi, en outre, que sur de l'oxyde de propylène, dans une proportion appropriée, en même temps ou dans un ordre quelconque.

19. Procédé selon la revendication 18, caractérisé en ce qu'on mélange le chitosane, en présence d'un solvant, avec l'halogénure de glycidyltrialkylammonium et l'oxyde de propylène et l'on mélange ou l'on agite ce mélange plusieurs heures à une température comprise entre 10 et 100°C.

20. Procédé selon les revendications 18 et 19, caractérisé en ce qu'on effectue la réaction en présence d'une base organique ou inorganique.

21 Procédé selon les revendications 18 et 19, caractérisé en ce qu'on effectue la réaction en présence d'un acide organique ou inorganique.

22. Procédé selon les revendications 18 à 21, caractérisé en ce qu'on utilise du chlorure de glycidyltriméthylammonium, comme halogénure de glycidyltrialkylammonium.

23. Procédé selon les revendications 18 à 22, caractérisé en ce qu'on utilise, comme matière de départ, du chitosane modifié structuralement par reprécipitation et congélation.

24. Procédé selon les revendications 18 à 23, caractérisé en ce qu'on effectue la réaction en présence d'eau